# EUROPÄISCHE PATENTSCHRIFT

(11) **EP 1 180 979 B1**
(45) Veröffentlichungstag und Bekanntmachung des Hinweises auf die Patenterteilung: **27.09.2006**
(21) Anmeldenummer: 99919014.3
(22) Anmeldetag: 14.05.1999
(51) Int. Cl.: A61B 17/70, A61B 17/80

(54) **KNOCHENFIXATIONSVORRICHTUNG MIT DREHGELENK**
BONE FIXATION DEVICE WITH A ROTATION JOINT
DISPOSITIF DE FIXATION OSSEUSE DOTE D'UNE ARTICULATION ROTOIDE

(43) Veröffentlichungstag der Anmeldung: 27.02.2002
(73) Patentinhaber: Synthes AG Chur, 7002 Chur (CH)
(72) Erfinder: SCHLÄPFER, Fridolin, J., CH-8750 Glarus (CH); HESS, Martin, CH-4434 Hölstein (CH)
(74) Vertreter: Lusuardi, Werther
(86) Internationale Anmeldenummer: PCT/CH1999/000203
(87) Internationale Veröffentlichungsnummer: WO 2000/069351

(56) Entgegenhaltungen:
- DE-A- 4 438 264
- FR-A- 2 726 459
- US-A- 5 380 324
- US-A- 5 470 333
- US-A- 5 707 372

## Beschreibung

Die Erfindung bezieht sich auf eine Vorrichtung zur Verbindung eines Körpers K_{A} mit einem Körper K_{B}, insbesondere von zwei Knochen oder Knochenteilen gemäss dem Oberbegriff des Patentanspruchs 1.

Bei Wirbelsäulendefekten im Bereich der lumbalen und thorakalen Wirbelsäule wird heute vermehrt von anterior operiert. Als Gründe für diesen Wechsel von posterioren Eingriffen zu anterioren Eingriffen sind zu nennen:
- Die Pathologie liegt oft im Bereich der anterioren Säule;
- Der anteriore Zugang zum Rückenmark ist einfacher;
- Die Dekompression des Rückenmarkes von anterior ist einfacher;
- Im Vergleich zum posterioren Zugang werden weniger muskuläre, neurologische und vaskuläre Strukturen zerstört; und
- Im Gegensatz zur postero-lateralen Fixation/Fusion ist eine Abstützung der anterioren Säule mechanisch logischer.

Ein internes anteriores Fixationssystem für die Behandlung von Wirbelsäulenfrakturen ist aus der WO 94/06360 DANEK bekannt. Dieses Fixationssystem umfasst eine längliche Platte mit einem integralen superioren, einem inferioren und einem dazwischen angeordneten Brückenabschnitt. Der superiore und der inferiore Abschnitt sind mit Mitteln zur Fixation an den entsprechenden Wirbel körpern versehen, während der Brückenabschnitt zwischen den anderen Abschnitten den verletzten Wirbelkörper überbrückt. Der superiore Abschnitt enthält zwei im wesentlichen parallele Schlitze mit Ausnehmungen zur Aufnahme von Knochenfixationsschrauben. Der inferiore Abschnitt enthält zwei Schraubenlöcher, worin die Knochenschrauben oder Bolzen befestigbar sind. Die Schlitze im superioren Abschnitt ermöglichen eine Kompression oder Distraktion der dazwischen liegenden Wirbelteile. Die Schraubenlöcher im inferioren Abschnitt weisen gegenüber der Plattenlängsachse einen Winkel auf, wodurch eine trapezoidartige Anordnung der Fixationsschrauben in der Platte entsteht. Je nach Ausführungsart dieses bekannten Fixationssystemes können die zwei posterior plazierten Knochenschrauben vor der Implantation der Knochenplatte gesetzt werden.

Die Knochenplatte wird nach dem Setzen dieser beiden Knochenschrauben durch einen schmalen Schlitz in den Körper geführt, auf die Knochenschrauben aufgesetzt und mit Muttern fixiert. Die zwei anterior plazierten Schrauben werden nach der Fixation der Knochenplatte durch die entsprechenden Schraubenlöcher in die Knochenplatte eingedreht.

Eine weitere Knochenplatte, welche eine Kontraktion oder Distraktion der zu verbindenden Knochenteile ermöglicht, ist aus der EP 0 829 240 HARMS bekannt. Diese bekannte Knochenplatte weist zwei gegeneinander in Richtung der Längsachse der Platte verschiebbare Abschnitte mit jeweils mindestens einer Bohrung für die Aufnahme von Knochenschrauben und dazwischen liegenden länglichen Mittelteilen auf. Die Fixation der beiden Abschnitte relativ zueinander wird durch zwei Schrauben, welche im Mittelteil des ersten Abschnittes in Bohrungen mit Innengewinde schraubbar sind und im Mittelteil des anderen Abschnittes durch zwei Langlöcher geführt werden, erreicht. Die Lange der gesamten Platte ist durch die beiden Langlöcher variabel gestaltbar. Der eine Abschnitt weist auf seiner intermediären Oberfläche eine Strukturierung auf, welche zur Arretierung der beiden Abschnitte relativ zueinander mittels eines durch eine entsprechende Ausnehmung am anderen Abschnitt führbaren Fixierelementes dient.

Nachteilig bei beiden bekannten Knochenfixationssystemen ist, dass:
a) Die Wirbel nicht über die Knochenschrauben manipuliert werden können, um iatrogene oder andere Fehlstellungen der Wirbel zu korrigieren;
b) Nur eine beschränkte Distraktions- beziehungsweise Kompressionsmöglichkeit besteht; und
c) Wenn das Implantat einmal gesetzt ist, keine in-situ Lordosier- beziehungsweise Kyphosiermöglichkeit mehr besteht.

Eine andere Vorrichtung zur Verbindung von Knochenfixationmitteln, welche ein um eine Achse drehbares Drehgelenk umfasst, ist aus der US 5,707,372 ERRICO bekannt.

Eine weitere, ebenfalls ein Drehgelenk umfassende Vorrichtung zur Verbindung von Knochenfixationsmitteln, insbesondere von Pedikelschrauben ist aus der US 5,470,333 RAY bekannt.

Bei beiden oben erwähnten Dokumenten dienen die Arretiermittel an den Drehgelenken simultan sowohl zur Blockierung der beiden Längsträger bezüglich Längsbewegungen relativ zueinander als auch zur Fixation des Drehgelenkes bei einem eingestellten Drehwinkel. Daher ist bei beiden Vorrichtung eine unabhängige Blockierung von Drehbewegung und Längsbewegung nicht möglich. Die entkoppelte Blockierung von Drehbewegung und Längsbewegung ist aber klinisch sehr wichtig.

Hier will die Erfindung Abhilfe schaffen. Der Erfindung liegt die Aufgabe zugrunde, eine Vorrichtung zur Fixation von Wirbelkörpern zu schaffen, welche eine Manipulation der Wirbel zur Korrektur von Fehlstellungen zulässt, eine kontinuierliche und sich über einen grossen Längenbereich erstreckende Längenverstellung ermöglicht und auch nach dem Setzen des Implantates eine in-situ Lordosier- beziehungsweise Kyphosiermöglichkeit gestattet.

Die Erfindung löst die gestellte Aufgabe mit einer Vorrichtung zur Verbindung eines Körpers K_{A} mit einem Körper K_{B}, insbesondere von zwei Knochen oder Knochenteilen, welche die Merkmale des Anspruchs 1 aufweist.

Weitere vorteilhafte Ausgestaltungen der Erfindung sind in den abhängigen Ansprüchen gekennzeichnet.

Die erfindungsgemässe Knochenfixationsvorrichtung umfasst eine kraniale und eine kaudale Knochenplatte, welche über je zwei Knochenschrauben fest mit den entsprechenden Wirbelkörpern verbindbar sind, und einen Längsträger, welcher die beiden Knochenplatten in einer zur Knochenoberfläche ungefähr parallelen Ebene drehbar verbindet. Die jeweils posterior plazierte Knochenschraube ist mittels eines durch eine konische Spannschraube elastisch spreizbaren, kugelartigen Schraubenkopfes unter verschiedenen Winkeln gegenüber der Bohrungsachse fixierbar. Anstelle des elastisch spreizbaren, kugelartigen Schraubenkopfes kann die jeweils posterior plazierte Knochenschraube auch mittels einer zwischen Knochenschraube und Knochenplatte angeordneten, kugelartigen Spannzange so fixiert werden, dass die Knochenschraube gegenüber der Bohrungsachse unter verschiedenen Winkeln blockierbar ist. Die jeweils anterior angeordnete Knochenschraube wird erst nach dem Setzen und Fixieren der Knochenplatte mit den posterior angeordneten Knochenschrauben eingesetzt. Der Winkel zwischen der Knochenplatte und der dazugehörenden anterior plazierten Knochenschraube ist durch die Bohrung in der Knochenplatte fest vorgegeben. Die Verbindung des Längsträgers mit der kaudalen Knochenplatte ist teleskopartig gestaltet, so dass eine winkelstabile Translation der kranialen Knochenplatte relativ zur kaudalen Knochenplatte ermöglicht wird. Der Längsträger ist mit einer oder mehreren Stellschrauben in der kaudalen Knochenplatte lösbar blockierbar. Die Verbindung zwischen kranialer Knochenplatte und Längsträger erfolgt über ein Drehgelenk mit einer im wesentlichen senkrecht zur Knochenoberfläche stehenden Drehachse und ist ebenfalls lösbar blockierbar. Mittels dieses Drehgelenkes ist der Winkel zwischen den beiden Knochenplatten einstellbar. Der so einstellbare Winkel liegt vorzugsweise in einem Bereich von +30° und -10°.
Translation und Rotation der Knochenplatten relativ zueinander sind unabhängig voneinander blockierbar. Zudem ist die kraniale Knochenplatte mit einem Innensechskant versehen, worüber ein Torsionsmoment auf die Knochenfixationsvorrichtung ausübbar ist.
Wenn das Drehgelenk nicht blockiert ist, kann durch das Torsionsmoment die Winkelstellung des kranialen Wirbels gegenüber dem kaudalen Wirbel korrigiert werden. Wenn das Drehgelenk blockiert ist und die kaudale anteriore Knochenschraube noch nicht gesetzt ist, kann durch das Torsionsmoment der kraniale Wirbel relativ zum kaudalen Wirbel anterior (Retrolisthesis) oder posterior (Anterolisthesis) verschoben werden.
Die Länge des Längsträgers ist so gestaltet, dass durch die erfindungsgemässe Knochenfixationsvorrichtung zwei aneinander angrenzende Wirbelkörper miteinander verbindbar sind. Bei entsprechender Wahl der Länge des Längsträgers lassen sich aber auch zwei Wirbelkörper miteinander verbinden, welche durch einen oder mehrere defekte Wirbelkörper getrennt sind.

Die erfindungsgemässe Knochenfixationsvorrichtung wird antero-lateral plaziert und kann mono- oder multisegmental eingesetzt werden. Die Fixationsvorrichtung kann je nach Bedarf mit posterioren Fixationssystemen (Knochenschrauben, Fixateur interne etc.) kombiniert werden, so dass sich bei Bedarf auch eine 360° - Fixation erstellen lässt.

Die erfindungsgemässe Knochenfixationsvorrichtung und die notwendigen Montageinstrumente sind derart konzipiert, dass sie endoskopisch oder durch offenen Zugang implantierbar ist.

Hauptindikationen der erfindungsgemässen Knochenfixationsvorrichtung sind Trauma, Tumore, Infekte, posttraumatische Kyphosen und Wirbeldegenerationen.

Das operative Vorgehen bei der Implantation der erfindungsgemässen Knochenfixationsvorrichtung erfolgt wie folgt:
1) Eindrehen der posterior zu plazierenden Knochenschrauben in die zu verbindenden Wirbelkörper. Dabei muss darauf geachtet werden, dass die Knochenschrauben möglichst weit posterior mit ungefähr gleichem Abstand von der hinteren Wand des entsprechenden Wirbels gesetzt und mehr oder weniger parallel zur hinteren Wand des entsprechenden Wirbels verlaufen. Die kaudale Knochenschraube wird nahe der kaudalen Deckplatte des kaudal zur Läsion gelegenen Wirbels gesetzt. Entsprechend wird die kraniale Knochenschraube nahe der kranialen Deckplatte des kranial zur Läsion gelegenen Wirbels positioniert;
2) Spreizen der Wirbelkörper mittels eines auf die Knochenschrauben aufgesetzten Spreizers. Damit lassen sich die Wirbelkörper auf Distanz bringen und die hintere Wand im Bereich der Läsion anatomisch rekonstruieren. Einsetzen eines entsprechenden Knochenspans;
3) Distanz zwischen den Knochenschrauben messen und entsprechende Fixationsvorrichtung auswählen. Die gewählte Fixationsvorrichtung auf die bereits eingedrehten Knochenschrauben aufsetzen (Drehgelenk und Teleskop lose). Bei skoliotischer Deformation im Bereich der Läsion kann über die Knochenschrauben die Deformation korrigiert werden. Anschliessend Blockierung der Winkelfreiheit der Knochenschrauben durch Blockieren der kugelförmigen Spannzangen;
4) Die einzelnen Knochenplatten derart ausrichten, dass sie parallel zur hinteren Wand des entsprechenden Wirbels stehen. Anterior zu plazierende Knochenschrauben eindrehen;

### Spezialfall:

Wenn der kraniale Wirbel gegenüber dem kaudalen Wirbel nach anterior oder posterior verschoben ist, darf nur die anterior plazierte Knochenschraube der kranialen Knochenplatte gesetzt werden. Die kaudale Knochenplatte ist nach wie vor nur über die posterior plazierte Knochenschraube mit dem kaudalen Wirbelkörper verbunden.

Für die Korrektur einer anterioren beziehungsweise posterioren Verschiebung des kranialen Wirbels relativ zum kaudalen Wirbel muss vorgängig das Drehgelenk blockiert werden. Anschliessend kann über den in der kranialen Knochenplatte angebrachten Innensechskant mittels eines entsprechenden Schraubendrehers ein Torsionsmoment aufgebracht werden, um den kranialen Wirbel nach anterior beziehungsweise posterior zu verschieben. Bei der Korrektur dreht sich die ganze Fixationsvorrichtung um die posterior plazierte Knochenschraube der kaudalen Knochenplatte.

Nach vollbrachter Korrektur wird nun auch die anterior plazierte Knochenschraube eingedreht. Mit dem Eindrehen der zweiten Knochenschraube in der kaudalen Knochenplatte ist die Korrektur fixiert und das Drehgelenk kann wieder gelöst werden, um die Winkelstellung des kranialen Wirbels relativ zum kaudalen Wirbel zu manipulieren.
5) Rekonstruktion der anatomischen Winkelstellung des kranialen Wirbels relativ zum kaudalen Wirbel über den in der kranialen Knochenplatte befindlichen Innensechskant und Blockierung des Drehgelenkes. Beim Aufrichten des kranialen Wirbels muss das Teleskop frei spielen können.
6) Kompression des Knochenspans und Blockierung des Teleskops.

Die durch die Erfindung erreichten Vorteile sind im wesentlichen darin zu sehen, dass dank der erfindungsgemässen Knochenfixationsvorrichtung:
a) Die Wirbel über die Knochenschrauben manipuliert werden können, um iatrogene oder andere Fehlstellungen der Wirbel zu korrigieren;
b) Eine kontinuierliche und sich über einem grossen Längenbereich erstreckende Längenverstellung möglich ist, so dass eine Distraktions- beziehungsweise Kompressionsmöglichkeit innerhalb eines grossen Bereiches besteht; und
c) Auch nach dem Setzen des Implantates eine in-situ Lordosier- beziehungsweise Kyphosiermöglichkeit besteht.

Die Erfindung und Weiterbildungen der Erfindung werden im folgenden anhand der teilweise schematischen Darstellungen mehrerer Ausführungsbeispiele noch näher erläutert.
Es zeigen:
Fig. 1 eine Ansicht von lateral auf einen Wirbelsäulenabschnitt mit einer Fixationsvorrichtung gemäss der Erfindung;
Fig. 2 einen Schnitt quer zur Wirbelsäulenlängsachse durch die bevorzugte Ausführungsform der kranialen Knochenplatte der erfindungsgemässen Vorrichtung im Bereich des Drehgelenkes;
Fig. 3 einen Schnitt quer zur Wirbelsäulenlängsachse durch eine weitere Ausführungsform der kranialen Knochenplatte der erfindungsgemässen Vorrichtung im Bereich des Drehgelenkes; und
Fig. 4. einen Schnitt quer zur Wirbelsäulenlängsachse durch eine nicht als Gegenstand der Erfindung zu betrachtende Ausführungsform der kranialen Knochenplatte im Bereich der Knochenschrauben.

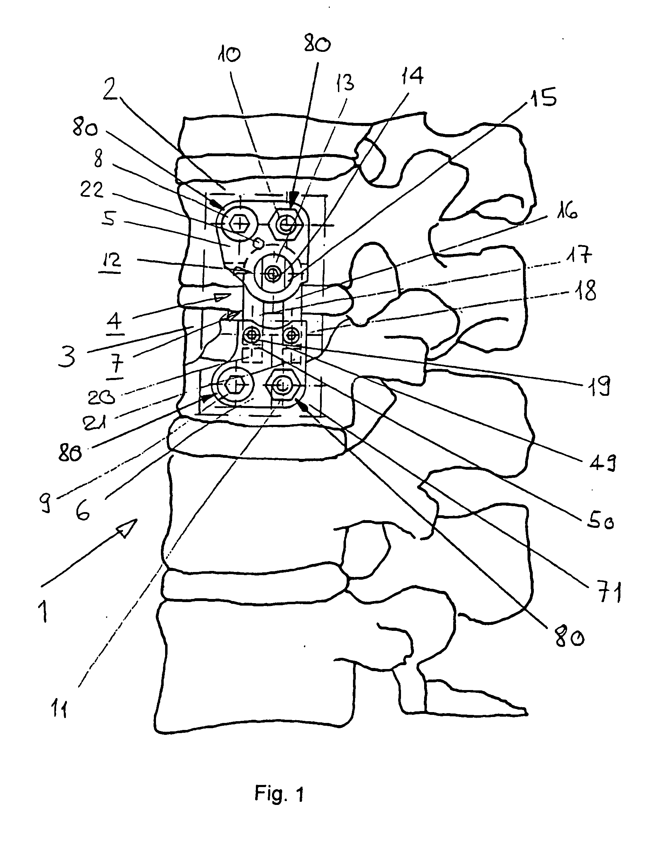

In Fig. 1 ist eine monosegmentale Anwendung der erfindungsgemässen Knochenfixationsvorrichtung 4 an einer Wirbelsäule 1 dargestellt. Durch die Knochenfixationsvorrichtung 4 wird ein kranialer Wirbel 2 mit einem partiell defekten kaudalen Wirbel 3 verbunden. Die Knochenfixationsvorrichtung 4 umfasst eine kraniale Knochenplatte 5, eine kaudale Knochenplatte 6 und einen Längsträger 7. Die kraniale sowie die kaudale Knochenplatte 5;6 weisen je eine an den Knochen angrenzende Unterseite 29 und eine Oberseite 52 auf, wobei die beiden Unterseiten der Knochenplatten 5;6 in einer gemeinsamen Fläche 71 liegen. Durch den Längsträger 7 sind die kraniale Knochenplatte 5 und die kaudale Knochenplatte 6 in parallel zur Wirbelssäulenlängsachse teleskopierbarer und mittels eines Gelenkes 12 in schwenkbarer Weise verbunden. Die Drehachse 27 des Gelenkes 12 steht im wesentlichen senkrecht zu der den Unterseiten 29 gemeinsamen Fläche 71. An den Wirbeln 2;3 befestigt werden die Knochenplatten 5;6 mit je einer anterior plazierten Knochenschraube 8;9 und je einer posterior plazierten Knochenschraube 10;11. Die Schwenkbarkeit der kranialen Knochenplatte 5 relativ zum Längsträger 7 wird durch ein Gelenk 12 erreicht. Das Gelenk 12 ist mittels eines Klemmkeiles 13 und einer Spannschraube 14 bezüglich Rotation fixierbar und verbindet den Längsträger 7 mit der kranialen Knochenplatte 5.
Zur Lösung oder Blockierung des Gelenkes 12 wird der Klemmkeil 13 mittels der Spannschraube 14 relativ zu der Büchse 15, welche auf dem in der kranialen Knochenplatte 5 gelagerten Abschnitt radial elastisch deformierbar ist, bewegt. Die Büchse 15 ist je nach Ausführungsform der erfindungsgemässen Vorrichtung mittels einer kraftschlüssigen oder einer formschlüssigen Verbindung mit dem Längsträger 7 verbunden. Beim Blockieren des Gelenkes 12 wird durch die Keilwirkung des Klemmkeiles 13 der elastisch deformierbare Abschnitt der Büchse 15 gegen die Bohrungswand in der kranialen Knochenplatte 5 gepresst, wodurch eine kraftschlüssige oder bei entsprechender Gestaltung der Büchse 15 und der kraniale Knochenplatte 5 eine formschlüssige Verbindung entsteht, wodurch das Gelenk 12 blockiert wird. Die Teleskopierbarkeit der Knochenfixationsvorrichtung 4 wird in dieser Ausführungsform der erfindungsgemässen Knochenfixationsvorrichtung 4 durch eine Ausführung des Längsträgers 7 mit zwei parallelen Stäben 16;17, welche in entsprechenden Führungskanälen 20;21 in der kaudalen Knochenplatte 6 in Wirbelsäulenlängsrichtung verschiebbar und mittels zwei Stellschrauben 18;19 fixierbar sind, erreicht.

Zudem ist die kraniale Knochenplatte 5 mit einem Innensechskant 22 versehen, worüber mittels eines darin eingesteckten Schraubendrehers ein Torsionsmoment auf die kraniale Knochenplatte 5 oder bei blockiertem Drehgelenk 12 auf die Knochenfixationsvorrichtung 4 ausübbar ist. Die Stäbe 16;17 sind an ihren in den Führungskanälen 20;21 der kaudalen Knochenplatte 6 eingeschobenen Enden mit Erhebungen 49;50 versehen, so dass der Längsträger 7 bei geöffneten Stellschrauben 18;19 nicht aus der kaudalen Knochenplatte 6 herausgleiten kann.

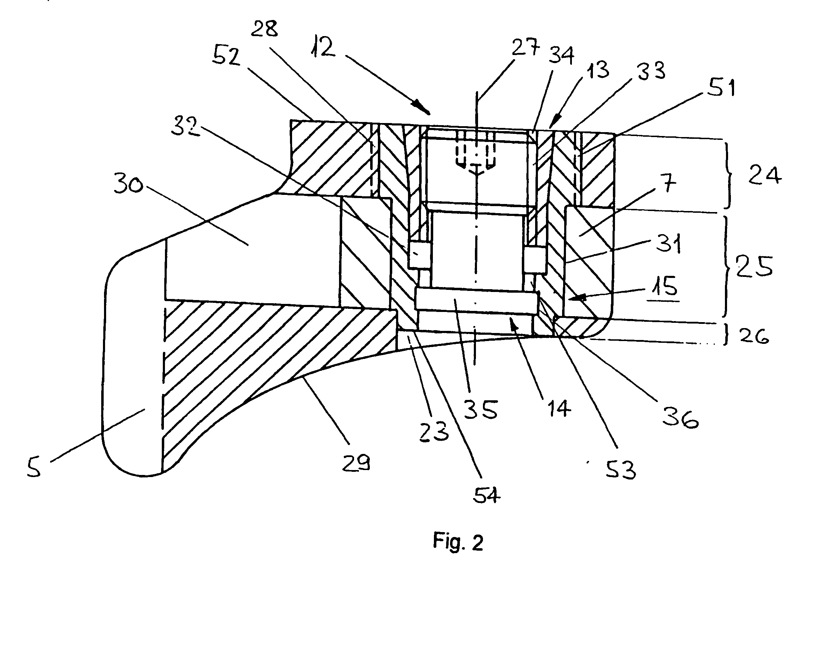

Fig. 2 zeigt einen quer zur Wirbelsäulenlängsachse verlaufenden Schnitt durch die kraniale Knochenplatte 5 und das Gelenk 12 der bevorzugten Ausführungsform der erfindungsgemässen Vorrichtung. Das Gelenk 12 umfasst eine zylindrische Büchse 15, einen Klemmkeil 13 und eine Spannschraube 14. Die kraniale Knochenplatte 5 ist auf ihrer dem kranialen Wirbelkörper 2 zugewandten Unterseite 29 konkav gestaltet und mit einer zu dieser Unterseite 29 im wesentlichen senkrechten Bohrung 23 mit der Zentralachse 27 ausgestattet. Eine die kraniale Knochenplatte 5 senkrecht zur Zentralachse 27 durchdringende Nut 30, welche gegen die kaudale Knochenplatte 6 hin offen ist, ermöglicht die Aufnahme des Längsträgers 7. Der Längsträger 7 ist konzentrisch zur Zentralachse 27 mit einer Bohrung 31 versehen, welche in der bevorzugten Ausführungsform so bemessen ist, dass zwischen dem mittleren Abschnitt 25 der Büchse 15 und der Bohrung 31 eine kraftschlüssige Verbindung mittels eines Presssitzes erfolgt. Eine formschlüssige Verbindung an dieser Stelle wäre mittels einer Nuten/Stift-Verbindung oder einer verzahnten Verbindung auch möglich. Im zwischen der Nut 30 und der Unterseite 29 liegenden Abschnitt der kranialen Knochenplatte 5 ist die Bohrung 23 so ausgeführt, dass der knochennahe Abschnitt 26 der Büchse 15 darin drehbar gelagert ist. Der knochenferne Abschnitt 24 der Büchse 15 ist im ebenfalls knochenfernen Teil der Bohrung 23 in der kranialen Knochenplatte 5 eingelassen, wobei der knochenferne Teil der Bohrung 23 mit einer Verzahnung 28 versehen ist, welche in ihrem Profil der Verzahnung 51 am knochenfernen Abschnitt 24 der Büchse 15 entspricht, so dass zwischen Büchse 15 und Bohrung 23 eine formschlüssige Verbindung vorliegt, wenn der Klemmkeil 13 so weit in den quer zur Zentralachse 27 verlaufenden, partiell keilförmig ausgebildeten Schlitz 32 eingetrieben ist, dass der knochenferne Abschnitt 24 der Büchse 15 so weit gespreizt wird, bis die Verzahnungen 28;51 ineinander eingreifen. Der Klemmkeil 13 selbst bildet eine in Bezug auf Rotation formschlüssige Verbindung zwischen der Büchse 15 und dem Längsträger 7. Bei gelöstem Klemmkeil 13 greifen die Verzahnungen 28;51 nicht ineinander ein, so dass die Büchse 15 in der Bohrung 23 mit dem Längsträger 7 frei um die Zentralachse 27 drehbar ist. Die Bewegung des Klemmkeiles 13 parallel zur Zentralachse 27 erfolgt mittels der Spannschraube 14, welche auf ihrem knochenfernen Teil mit einem Aussengewinde 33 und auf dem knochennahen Teil mit einem Wulst 35 versehen ist. Entsprechend zur Spannschraube 14 sind im Klemmkeil 13 ein Innengewinde 34 und in der Büchse 15 eine Ringnute 36 angebracht. Die Ringnute 36 kann nach dem Einführen der Spannschraube 14 in die Bohrung 53 der Büchse 15 durch Einwalzen des knochennahen Endes 54 der Büchse 15 erzeugt werden.

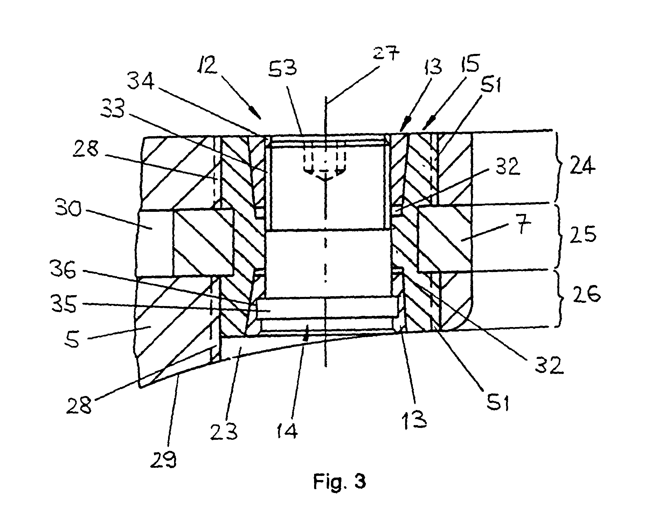

In Fig. 3 ist eine andere Ausführungsform des Gelenkes 12 der erfindungsgemässen Vorrichtung dargestellt. Diese Ausführungsform des Gelenkes 12 unterscheidet sich von der in Fig. 2 dargestellten Ausführungsform nur darin, dass die Büchse 15 auch an ihrem knochennahen Abschnitt 26 aussen mit einer Verzahnung 51 und einem quer zur Zentralachse 27 verlaufenden, partiell keilförmigen Schlitz 32 versehen ist. Der knochennahe Teil der Bohrung ist so mit einer zur Verzahnung 51 korrespondierenden Verzahnung 28 ausgestattet, dass zwischen Büchse 15 und Bohrung 23 eine formschlüssige Verbindung vorliegt, wenn der zweite am knochennahen Abschnitt 26 der Büchse 15 in den Schlitz 32 einpressbare Keil 13 so weit in diesen Schlitz 32 eingetrieben wird, dass der knochennahe Abschnitt 26 der Büchse 15 so weit gespreizt wird, bis die Verzahnungen 28;51 ineinander eingreifen. Die Klemmkeile 13 selbst bilden eine in Bezug auf Rotation formschlüssige Verbindung zwischen der Büchse 15 und dem Längsträger 7. Bei gelöstem Klemmkeil 13 greifen die Verzahnungen 28;51 nicht ineinander ein, so dass die Büchse 15 in der Bohrung 23 frei um die Zentralachse 27 drehbar ist. Die Bewegung der Klemmkeile 13 parallel zur Zentralachse 27 erfolgt mittels der Spannschraube 14, welche auf ihrem knochenfernen Teil mit einem Aussengewinde 33 und auf dem knochennahen Teil mit einem Wulst 35 versehen ist. Entsprechend zur Spannschraube 14 sind in dem Klemmkeil 13, welcher im knochenfernen Abschnitt 24 der Büchse 15 eingefügt ist, ein Innengewinde 34 und in dem Klemmkeil 13, welcher im knochennahen Abschnitt 26 der Büchse 15 eingefügt ist, eine Ringnute 36 angebracht. Diese Anordnung der Klemmkeile 13, der Spannschraube 14 und der Büchse 15 gestattet, dass beim Anziehen der Spannschraube 14 beide Klemmkeile 13 gegeneinander verschoben werden, so dass die beiden elastisch spreizbaren Abschnitte 24;26 der Büchse 15 gegen die Wand der Bohrung 23 und somit die Aussenverzahnungen 28 an der Büchse 15 gegen die Innenverzahnungen 51 in der Bohrung 23 gepresst werden. Beim Lösen der Spannschraube 14 werden die Klemmkeile 13 von einander weg verschoben, wodurch die beiden elastisch deformierbaren Abschnitte 24;26 der Büchse 15 zurückfedern, bis die Verzahnungen 28;51 nicht mehr ineinander eingreifen, so dass die Büchse 15 in der Bohrung 23 frei um die Zentralachse 27 drehbar ist. Die Ringnute 36 kann nach dem Einführen der Spannschraube 14 in die Bohrung 53 der Büchse 15 durch Einwalzen der Wand des knochennahen Klemmkeils 13 erzeugt werden.

In einer weiteren Ausführungsform der erfindungsgemässen Vorrichtung (nicht gezeichnet) ist die frei drehbare, aber axialfeste Verbindung zwischen Spannschraube 14 und Büchse 15 durch den in der Nute 36 gelagerten Wulst 35 durch eine frei drehbare, aber axialfeste Verbindung zwischen Spannschraube 14 und knochennahem Klemmkeil 13 ersetzt. Der Wulst 35 an der Spannschraube 14 und eine ringförmige Nute im knochennahen Klemmkeil 13 sind anstelle der Gewindeverbindung zwischen dem Aussen-Rechtsgewinde 56 an der Spannschraube 14 und dem Innen-Rechtsgewinde 55 am knochennahen Klemmkeil 13 angebracht.

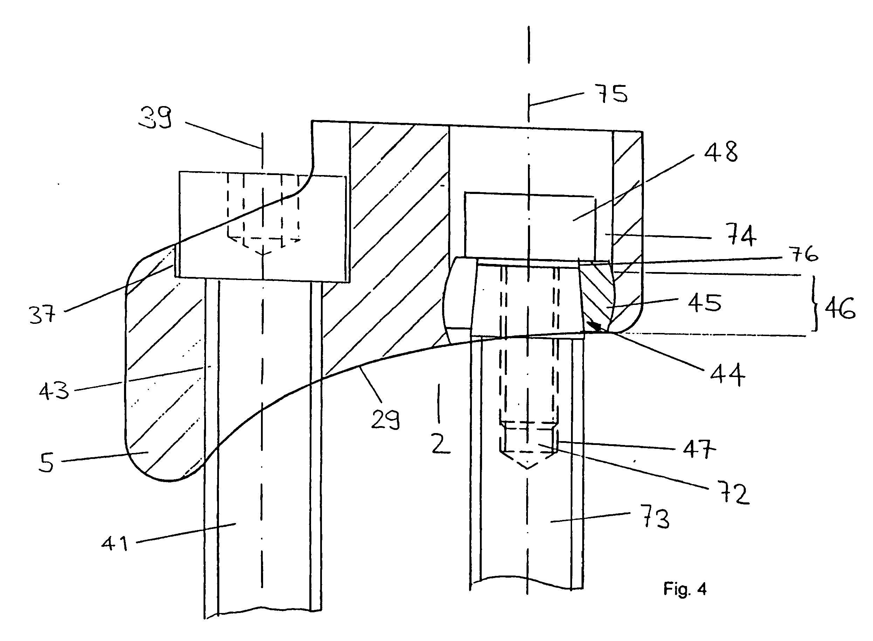

In Fig. 4 ist ein quer zur Wirbelsäulenlängsachse verlaufenden Schnitt durch die kraniale Knochenplatte 5 und die Bohrungen 37;74 zur Aufnahme der Knochenschrauben 41;73. Die Längsachsen 39;75 der Bohrungen 37;74 verlaufen im wesentlich senkrecht zur an die Oberfläche des Wirbelkörpers 2 angrenzenden Unterseite 29 der kranialen Knochenplatte 5. Während die anterior plazierte Knochenschraube 41 in eine mit einem Innengewinde 43 versehene Bohrung 37 in der kranialen Knochenplatte 5 in den kranialen Wirbelkörper 2 eingeschraubt wird, wird die posterior plazierte Knochenschraube 73 mittels einer Konusverbindung 44 in einer kugelschichtförmigen Spannzange 45 befestigt. Die Spannzange 45 ist in einem kugelartigen Abschnitt 46 in der Bohrung 74 axialfest und um das Kugelzentrum schwenkbar gelagert. Die posterior plazierte Knochenschraube 73 wird mittels einer Schraube 48 fixiert, welche in das Innengewinde 47 schraubbar ist, das in der Bohrung 72 am knochenfernen Ende der Knochenschraube 73 angebracht ist. Bei der Fixation dieser posterior plazierten Knochenschraube 73 wird die Spannzange 45 beim Anziehen der Schraube 48 durch die Keilwirkung der Konusverbindung 44 expandiert und gegen die Wand des kugelartigen Abschnittes 46 der Bohrung 74 gepresst. Durch diese Ausgestaltung lässt sich erreichen, dass die posterior plazierte Knochenschraube 73 auch winklig gegenüber der Längsachse 75 fixierbar ist.

Die Anordnung der Bohrungen 37;38;74 ist für die kaudale Knochenplatte 6 spiegelbildlich.

Bei den in den Fig. 2 und 3 dargestellten Ausführungsformen der erfindungsgemässen Vorrichtung können anstelle von Klemmkeilen 13 auch Klemmkonusse eingesetzt werden. Die Ausnehmung 32 in der Büchse 15 ist dann entsprechend zu gestalten.

## Patentansprüche

1. Vorrichtung zur Verbindung eines Körpers K_{A} mit einem Körper K_{B}, insbesondere von zwei Knochen oder Knochenteilen, mit
A) zwei Platten P_{A};P_{B} (5;6), welche je eine einem zu verbindenden Körper K_{A};K_{B} zugewandte Unterseite (29), eine Oberseite (52) und je mindestens eine die Platte P_{A};P_{B} (5;6) in einem Winkel zwischen 60° und 90° zur Unterseite (29) durchdringende Bohrung (37;38) zur Aufnahme eines Verankerungselementes (80) aufweisen, mittels welchem jede der Platten P_{A};P_{B} (5;6) an einem entsprechenden Körper K_{A}; K_{B} befestigbar ist;
B) einem Längsträger (7), mittels welchem die beiden Platten P_{A};P_{B} (5;6) so verbindbar sind, dass die Unterseiten (29) der Platten P_{A};P_{B} (5;6) in einer gemeinsamen Fläche (71) liegen und der Abstand zwischen den beiden Platten P_{A};P_{B} (5;6) parallel zur gemeinsamen Fläche (71) verstellbar ist, wobei
C) der Längsträger (7) über ein lösbar blockierbares Gelenk (12) mit einer der Platten P_{A};P_{B} (5;6) verbunden ist,
**dadurch gekennzeichnet, dass**
D) das Gelenk (12) als Schamiergelenk ausgebildet ist, und eine auf mindestens einem Teil ihrer Länge elastisch spreizbare Büchse (15), mindestens ein Klemmelement (13) und ein Spannmittel (14) umfasst, wobei das mindestens eine Klemmelement (13) mittels dem Spannmittel (14) relativ zur Büchse (15) bewegbar ist.

2. Vorrichtung nach Anspruch 1, **dadurch gekennzeichnet, dass** der Längsträger (7) einseitig mit einer der Platten P_{A};P_{B} (5;6) über ein lösbar blockierbares Gelenk (12) verbunden ist.

3. Vorrichtung nach Anspruch 2, **dadurch gekennzeichnet, dass** die Verbindung zwischen dem Längsträger (7) und einer der Platten P_{A};P_{B} (5;6) eine Verschiebung zwischen Längsträger (7) und der Platte P_{A};P_{B} (5;6) zulässt und die Verbindung lösbar blockierbar ist.

4. Vorrichtung nach Anspruch 1 oder 3, **dadurch gekennzeichnet, dass** der Längsträger (7) mit jeder der Platten P_{A};P_{B} (5;6) über ein lösbar blockierbares Gelenk (12) verbunden ist.

5. Vorrichtung nach einem der Ansprüche 1 bis 4, **dadurch gekennzeichnet, dass** der Längsträger (7) selbst teleskopierbar ist, wobei die Lange des Längsträgers (7) lösbar fixierbar ist.

6. Vorrichtung nach einem der Ansprüche 1 bis 5, **dadurch gekennzeichnet, dass** das Gelenk (12) ein Scharniergelenk mit einer im wesentlichen senkrecht zur Unterseite (29) stehenden Drehachse (27) ist.

7. Vorrichtung nach Anspruch 6, **dadurch gekennzeichnet, dass** das Gelenk (12) eine im Längsträger (7) verankerte Welle (57) umfasst, welche in einer in Platte P_{A} respektive P_{B} (5;6) konzentrisch zur Drehachse (27) angebrachten Bohrung (23) drehbar gelagert ist.

8. Vorrichtung nach Anspruch 7, **dadurch gekennzeichnet, dass** die Welle (57) kraftschlüssig im Längsträger (7) verankert ist.

9. Vorrichtung nach Anspruch 7, **dadurch gekennzeichnet, dass** die Welle (57) formschlüssig im Längsträger (7) verankert ist.

10. Vorrichtung nach Anspruch 6, **dadurch gekennzeichnet, dass** das Gelenk (12) eine in der Platte P_{A} respektive P_{B} (5;6) konzentrisch zur Drehachse (27) verankerte Welle (57) umfasst, welche in einer im Längsträger (7) konzentrisch zur Drehachse (27) angebrachten Bohrung (31) drehbar gelagert ist.

11. Vorrichtung nach Anspruch 10, **dadurch gekennzeichnet, dass** die Welle (57) kraftschlüssig in der Platte P_{A} respektive P_{B} (5;6) verankert ist.

12. Vorrichtung nach Anspruch 10, **dadurch gekennzeichnet, dass** die Welle (57) formschlüssig in der Platte P_{A} respektive P_{B} (5;6) verankert ist.

13. Vorrichtung nach Anspruch 6, **dadurch gekennzeichnet, dass** das Gelenk (12) eine konzentrisch zur Drehachse (27) angeordnete Welle (57) umfasst, welche in der Platte P_{A} respektive P_{B} (5;6) und dem Längsträger (7) drehbar gelagert ist.

14. Vorrichtung nach einem der Ansprüche 7 bis 13, **dadurch gekennzeichnet, dass** die Welle (57) als Büchse (15) ausgeführt ist, wobei die Büchse (15) über einen gewissen Bereich ihrer Lange elastisch spreizbar ist.

15. Vorrichtung nach Anspruch 14, **dadurch gekennzeichnet, dass** ein Klemmelement (13) und ein Spannmittel (14) in die Büchse (15) integriert sind, wobei das Klemmelement (13) mittels dem Spannmittel (14) relativ zur Büchse (15) parallel zur Drehachse (27) bewegbar ist, so dass die Büchse (15) auf Grund der Relativbewegung des Klemmelementes (13) gespreizt wird.

16. Vorrichtung nach Anspruch 15, **dadurch gekennzeichnet, dass** das Klemmelement (13) bezüglich Rotation eine formschlüssige Verbindung zwischen dem Längsträger (7) und der Büchse (15) bildet und bezüglich Verschiebung längs der Drehachse (27) frei beweglich ist.

17. Vorrichtung nach Anspruch 15, **dadurch gekennzeichnet, dass** das Klemmelement (13) bezüglich Rotation eine formschlüssige Verbindung zwischen der Platte P_{A} resp. P_{B} (5;6) und der Büchse (15) bildet und bezüglich Verschiebung längs der Drehachse (27) frei beweglich ist.

18. Vorrichtung nach Anspruch 14, **dadurch gekennzeichnet, dass** zwei Klemmelemente (13) und ein Spannmittel (14) in die Büchse (15) integriert sind, wobei die Klemmelemente (13) mittels des Spannmittels (14) längs der Drehachse (27) relativ zueinander bewegbar sind und die Büchse (15) auf Grund dieser Bewegung der Klemmelemente (13) spreizbar ist.

19. Vorrichtung nach Anspruch 18, **dadurch gekennzeichnet, dass** mindestens eines der Klemmelemente (13) bezüglich Rotation eine formschlüssige Verbindung zwischen dem Längsträger (7) und der Büchse (15) bildet und bezüglich Verschiebung längs der Drehachse (27) frei beweglich ist.

20. Vorrichtung nach Anspruch 18, **dadurch gekennzeichnet, dass** mindestens eines der Klemmelemente (13) bezüglich Rotation eine formschlüssige Verbindung zwischen der Platte P_{A} resp. P_{B} (5;6) und der Büchse (15) bildet und bezüglich Verschiebung längs der Drehachse (27) frei beweglich ist.

21. Vorrichtung nach einem der Ansprüche 15 bis 20, **dadurch gekennzeichnet, dass** die Büchse (15) im spreizbaren Bereich längs der Drehachse (27) geschlitzt ist.

22. Vorrichtung nach einem der Ansprüche 15 bis 21, **dadurch gekennzeichnet, dass** das oder die Klemmelemente (13) mindestens partiell keilförmig ausgebildet sind.

23. Vorrichtung nach einem der Ansprüche 15 bis 22, **dadurch gekennzeichnet, dass** die Büchse (15) mindestens partiell mit einem keilförmigen Schlitz (32) versehen ist.

24. Vorrichtung nach den Ansprüchen 22 und 23, **dadurch gekennzeichnet, dass** die Keilformen des Schlitzes (32) und des Klemmelementes (13) komplementär ausgebildet sind.

25. Vorrichtung nach einem der Ansprüche 22 bis 24, **dadurch gekennzeichnet, dass** die Keilformen des Schlitzes (32) und des Klemmelementes (13) einen halben Keilwinkel α zwischen 3° und 20° aufweisen.

26. Vorrichtung nach Anspruch 25, **dadurch gekennzeichnet, dass** der halbe Keilwinkel α zwischen 5° und 10° beträgt.

27. Vorrichtung nach einem der Ansprüche 15 bis 26, **dadurch gekennzeichnet, dass** das Spannmittel (14) ein Gewindebolzen ist.

28. Vorrichtung nach Anspruch 27, **dadurch gekennzeichnet, dass** der Gewindebolzen ein Gewinde (33) aufweist.

29. Vorrichtung nach den Ansprüchen 15 und 28, **dadurch gekennzeichnet, dass** das Klemmelement (13) ein zum Gewinde (33) des Gewindebolzens komplementäres Innengewinde (34) aufweist und der Gewindebolzen drehbar aber axial fest in der Büchse (15) gelagert ist.

30. Vorrichtung nach den Ansprüchen 15 und 28, **dadurch gekennzeichnet, dass** die Büchse (15) ein zum Gewinde (33) komplementäres Innengewinde aufweist und der Gewindebolzen derart im Klemmelement (13) gelagert ist, dass er relativ zum Klemmelement (13) drehbar aber nicht axial verschiebbar ist.

31. Vorrichtung nach den Ansprüchen 18 und 28, **dadurch gekennzeichnet, dass** das eine Klemmelement (13) ein zum Gewinde (33) des Gewindebolzens komplementäres Innengewinde aufweist und der Gewindebolzen derart im zweiten Klemmelement (13) gelagert ist, dass der Gewindebolzen relativ zum betreffenden Klemmelement (13) drehbar aber nicht axial verschiebbar ist und beim Drehen des Gewindebolzens sich die beiden Klemmelemente (13) relativ zueinander längs der Drehachse (27) verschieben.

32. Vorrichtung nach einem der Ansprüche 1 bis 31, **dadurch gekennzeichnet, dass** beim Verschieben des Klemmelementes (13) die Büchse (15) im spreizbaren Bereich so weit expandiert wird, dass eine kraftschlüssige Verbindung zwischen Büchse (15) und der umgebenden Bohrungswand entsteht.

33. Vorrichtung nach einem der Ansprüche 1 bis 31, **dadurch gekennzeichnet, dass** beim Verschieben des Klemmelementes (13) die Büchse (15) im spreizbaren Bereich so weit expandiert wird, dass eine formschlüssige Verbindung zwischen Büchse (15) und der umgebenden Bohrungswand entsteht.

34. Vorrichtung nach Anspruch 33, **dadurch gekennzeichnet, dass** die formschlüssige Verbindung aus komplementären Verzahnungen (28;51) besteht, welche an dem elastisch spreizbaren Bereich der Büchse (15) und entsprechend an der die Büchse (15) umgebenden Bohrungswand angebracht sind.

35. Vorrichtung nach Anspruch 34, **dadurch gekennzeichnet, dass** die Verzahnungen (28;51) einen Teilungswinkel zwischen 2° und 6° aufweisen.

36. Vorrichtung nach Anspruch 35, **dadurch gekennzeichnet, dass** die Verzahnungen (28;51) einen Teilungswinkel zwischen 2,5° und 3,5° aufweisen.

37. Vorrichtung nach Anspruch 35 oder 36, **dadurch gekennzeichnet, dass** die Zahnspitzen der Verzahnungen (28;51) einen Winkel zwischen 60° und 140° aufweisen.

38. Vorrichtung nach Anspruch 37, **dadurch gekennzeichnet, dass** die Zahnspitzen der Verzahnungen (28;51) einen Winkel zwischen 90° und 120° aufweisen.

39. Vorrichtung nach einem der Ansprüche 1 bis 38, **dadurch gekennzeichnet, dass** die Abstandverstellung zwischen den Platten P_{A} (5) und P_{B} (6) teleskopartig ist.

40. Vorrichtung nach einem der Ansprüche 1 bis 39, **dadurch gekennzeichnet, dass** die über das Drehgelenk (12) mit dem Längsträger (7) verbundene Platte P_{A} respektive P_{B} (5;6) eine Ausnahme (22) aufweist, über die mit einem Werkzeug ein Torsionsmoment um eine Achse im wesentlichen senkrecht zur Unterseite (29) auf die Vorrichtung ausübbar ist.

41. Vorrichtung nach einem der Ansprüche 1 bis 40, **dadurch gekennzeichnet, dass** der Längsträger (7) eine Ausnahme aufweist, über die mit einem Werkzeug ein Torsionsmoment um eine Achse im wesentlichen senkrecht zur durch die Unterseiten (29) der Platten P_{A};P_{B} (5;6) aufgespannten Fläche (71) auf die Vorrichtung ausübbar ist.

42. Vorrichtung nach einem der Ansprüche 1 bis 39, **dadurch gekennzeichnet, dass** die Platte P_{A} (5) und die Platte P_{B} (6) je eine Ausnahme aufweist, über die mit einem Werkzeug ein Torsionsmoment um eine Achse im wesentlichen senkrecht zur durch die Unterseiten (29) der Platten P_{A};P_{B} (5;6) aufgespannten Fläche (71) auf die Vorrichtung ausübbar ist.

43. Vorrichtung nach einem der Ansprüche 1 bis 42, **dadurch gekennzeichnet, dass** sie zusätzlich mindestens zwei Knochenschrauben (8;9;10;11;41;42;73) umfasst und jede der Platten P_{A};P_{B} (5;6) über mindestens eine Knochenschraube (8;9;10;11;41;42;73) mit einem Körper K_{A};K_{B} verbindbar ist.

44. Vorrichtung nach einem der Ansprüche 1 bis 43, **dadurch gekennzeichnet, dass** sie zusätzlich mindestens zwei Knochenschrauben (8;9;10;11;41;42;73) umfasst und pro Platte P_{A};P_{B} (5;6) mindestens eine Knochenschraube (42;73) in einem beliebigen Winkel bis zu ± 20° zur Senkrechten auf die Unterseite (29) lösbar blockierbar ist.

45. Vorrichtung nach Anspruch 44, **dadurch gekennzeichnet, dass** pro Platte P_{A}; P_{B} (5;6) mindestens eine Knochenschraube (42;73) in einem beliebigen Winkel zwischen ± 15° und ± 18° zur Senkrechten auf die Unterseite (29) lösbar blockierbar ist.

46. Vorrichtung nach Anspruch 45, **dadurch gekennzeichnet, dass** die Durchgangsbohrung (38;74) für die winklig einstellbare Knochenschraube (42) in den Platten P_{A};P_{B} (5;6) einen kalottenförmig ausgebildeten Abschnitt (46) aufweist.

47. Vorrichtung nach Anspruch 46, **dadurch gekennzeichnet, dass** der kalottenförmige Abschnitt (46) der Durchgangsbohrung (38) eine aufgerauhte Oberfläche aufweist.

48. Vorrichtung nach Anspruch 46, **dadurch gekennzeichnet, dass** der kalottenförmige Abschnitt (46) der Durchgangsbohrung (38) eine strukturierte Oberfläche aufweist.

49. Vorrichtung nach Anspruch 46, **dadurch gekennzeichnet, dass** der kalottenförmige Abschnitt (46) der Durchgangsbohrung (38) eine verzahnte Oberfläche aufweist.

50. Vorrichtung nach einem der Ansprüche 46 bis 49, **dadurch gekennzeichnet, dass** die Knochenschraube (42) einen kugelschichtförmigen, geschlitzten und radial elastisch deformierbaren Schraubenkopf (62) aufweist.

51. Vorrichtung nach Anspruch 50, **dadurch gekennzeichnet, dass** der kugelschichtförmige Schraubenkopf (62) der Knochenschraube (42) eine Aufnahme für ein Spannelement (66) zum Spreizen des kugelschichtförmigen geschlitzten Schraubenkopfes (62) aufweist.

52. Vorrichtung nach Anspruch 51, **dadurch gekennzeichnet, dass** die Aufnahme aus einem gegen die Öffnung hin divergierenden Innenkonus und einem in der Tiefe anschliessenden Innengewinde besteht und das Spannelement eine Schraube mit einem zum Innengewinde komplementären Aussengewinde und einem zum Innenkonus komplementären Aussenkonus ist.

53. Vorrichtung nach Anspruch 51, **dadurch gekennzeichnet, dass** die Aufnahme aus einem gegen die Öffnung hin divergierenden konischen Innengewinde (64) besteht und das Spannelement (66) eine Schraube mit einem zum Innengewinde (64) komplementären Aussengewinde ist.

54. Vorrichtung nach einem der Ansprüche 46 bis 49, **dadurch gekennzeichnet, dass** eine kugelschichtförmige geschlitzte Spannzange (45) in den kalottenförmigen Abschnitt (46) integriert ist.

55. Vorrichtung nach Anspruch 54, **dadurch gekennzeichnet, dass** die kugelschichtförmige Spannzange (45) im Vergleich zur Platte P_{A};P_{B} (5;6) aus einem weicheren Material besteht.

56. Vorrichtung nach Anspruch 54, **dadurch gekennzeichnet, dass** die Spannzange (45) eine strukturierte Oberfläche aufweist.

57. Vorrichtung nach Anspruch 56, **dadurch gekennzeichnet, dass** die Spannzange (45) eine verzahnte Oberfläche aufweist.

58. Vorrichtung nach Anspruch 54, **dadurch gekennzeichnet, dass** die Spannzange (45) eine aufgerauhte Oberfläche aufweist.

59. Vorrichtung nach einem der Ansprüche 54 bis 58, **dadurch gekennzeichnet, dass** die Spannzange (45) eine in Richtung der Längsachse (75) der Knochenschraube (73) verlaufende, gegen die Unterseite (29) hin konisch divergierende Durchgangsbohrung (76) aufweist, wobei der halbe Konuswinkel zwischen 3° und 10° beträgt.

60. Vorrichtung nach Anspruch 59, **dadurch gekennzeichnet, dass** der halbe Konuswinkel zwischen 3° und 5° beträgt.

61. Vorrichtung nach Anspruch 59 oder 60, **dadurch gekennzeichnet, dass** die Knochenschraube (73) im Bereich der Spannzange (45) einen im wesentlichen komplementären Aussenkonus aufweist.

62. Vorrichtung nach Anspruch 61, **dadurch gekennzeichnet, dass** die Knochenschraube (73) am Ende des Aussenkonus eine Aufnahme für ein Spannmittel zur Verspannung des Aussenkonus im Innenkonus der Spannzange (45) aufweist.

63. Vorrichtung nach Anspruch 62, **dadurch gekennzeichnet, dass** die Aufnahme im wesentlichen aus einem Innengewinde (47) und das Spannmittel aus einer Schraube (48) mit einem zum Innengewinde (47) komplementären Aussengewinde und einem Kopfdurchmesser grösser als der kleinste Durchmesser des Innenkonus der Spannzange (45) besteht.

64. Vorrichtung nach Anspruch 62, **dadurch gekennzeichnet, dass** die Aufnahme im wesentlichen aus einem Aussengewinde und das Spannmittel aus einer Mutter mit einem zum Aussengewinde komplementären Innengewinde und einem Aussendurchmesser grösser als der kleinste Durchmesser des Innenkonus der Spannzange (45) besteht.

65. Vorrichtung nach Anspruch 61, **dadurch gekennzeichnet, dass** die Spannzange (45) ein Innengewinde und die Knochenschraube (73) ein komplementäres Aussengewinde aufweist.

66. Vorrichtung nach einem der Ansprüche 43 bis 65, **dadurch gekennzeichnet, dass** pro Platte P_{A};P_{B} (5;6) mindestens eine Knochenschraube (41) in einem vorgegebenen Winkel zwischen 70° und 90° zur Unterseite (29) der Platten P_{A};P_{B} (5;6) lösbar blockierbar ist.

67. Vorrichtung nach Anspruch 66, **dadurch gekennzeichnet, dass** pro Platte P_{A};P_{B} (5;6) mindestens eine Knochenschraube (41) in einem vorgegebenen Winkel zwischen 80° und 90° zur Unterseite (29) der Platten P_{A};P_{B} (5;6) lösbar blockierbar ist.

68. Vorrichtung nach Anspruch 66 oder 67, **dadurch gekennzeichnet, dass** die in einem vorgegebenen Winkel zur Platte P_{A};P_{B} (5;6) einführbare Knochenschraube (41) im Kontaktbereich mit der Platte P_{A};P_{B} (5;6) ein Aussengewinde aufweist, das gegen das komplementäre, sich in der Bohrung (37) der Platte P_{A};P_{B} (5;6) befindliche Innengewinde (43) verspannt wird.

69. Vorrichtung nach einem der Ansprüche 43 bis 68, **dadurch gekennzeichnet, dass** pro Platte P_{A};P_{B} (5;6) mindestens eine Knochenschraube (42) in einem beliebigen Winkel zwischen 60° und 90° zur Unterseite (29) einbringbar ist.

70. Vorrichtung nach Anspruch 69, **dadurch gekennzeichnet, dass** pro Platte P_{A};P_{B} (5;6) mindestens eine Knochenschraube (42) in einem beliebigen Winkel zwischen 70° und 90° zur Unterseite (29) einbringbar ist.

71. Vorrichtung nach einem der Ansprüche 15 bis 21 und 27 bis 70, **dadurch gekennzeichnet, dass** das oder die Klemmelemente (13) mindestens partiell konusförmig ausgebildet sind.

72. Vorrichtung nach einem der Ansprüche 15 bis 21 und 27 bis 71, **dadurch gekennzeichnet, dass** die Büchse (15) mindestens partiell mit einem Innenkonus versehen ist.

73. Vorrichtung nach den Ansprüchen 71 und 72, **dadurch gekennzeichnet, dass** die Konusformen in der Büchse (15) und des Klemmelementes (13) komplementär ausgebildet sind.

74. Vorrichtung nach einem der Ansprüche 71 bis 73, **dadurch gekennzeichnet, dass** die Konusformen in der Büchse (15) und des Klemmelementes (13) einen halben Konuswinkel β zwischen 3° und 20 ° aufweisen.

75. Vorrichtung nach Anspruch 74, **dadurch gekennzeichnet, dass** der halbe Konuswinkel β zwischen 5° und 10° beträgt.

76. Vorrichtung nach Anspruch 1, **dadurch gekennzeichnet, dass** die Büchse (15) im Längsträger (7) verankert ist.

77. Vorrichtung nach Anspruch 1 , **dadurch gekennzeichnet, dass** der Längsträger (7) mit der anderen Platte P_{A};P_{B} (5;6) teleskopartig verschiebbar und winkelstabil verbunden ist.

## Claims

1. A device for connecting a body K_{A} with a body K_{B}, in particular two bones or bone parts, including
A) two plates P_{A};P_{B} (5;6) having each a bottom surface (29) adjoining a body K_{A};K_{B} to be connected, a top surface (52), and at least one bore (37;38) penetrating the plate P_{A};P_{B} (5;6) at an angle of between 60 and 90 degrees relative to the bottom surface (29) for receiving an anchoring member (80) by means of which each of the plates P_{A};P_{B} (5;6) is fastenable to a body K_{A};K_{B};
B) a longitudinal carrier (7) by means of which the two plates P_{A};P_{B} (5;6) are connectable in such a way that the bottom surfaces (29) of the plates P_{A};P_{B} (5;6) lie in a common surface (71) and that the distance between the two plates P_{A};P_{B} (5;6) is adjustable parallel to the common surface (71), whereby
C) the longitudinal carrier (7) is connected to one of the plates P_{A};P_{B} (5;6) by means of a releasably lockable joint (12),
**characterised in that**
D) the joint (12) is configured as a hinge joint, and comprises a sleeve (15) being capable of being resiliently spread apart over at least a part of ist length, at least one clamping member (13) and a straining means (14), whereby the at least one clamping member (13) is displaceable relative to the sleeve (15) by means of the straining member (14).

2. A device as claimed in claim 1, **characterised in that** the longitudinal carrier (7) is connected on one side with one of the plates P_{A};P_{B} (5;6) by means of a releasably lockable joint (12).

3. A device as claimed in claim 2, **characterised in that** the connection between the longitudinal carrier (7) and one of the plates P_{A};P_{B} (5;6) allows the longitudinal carrier (7) and the respective plate P_{A};P_{B} (5;6) to be displaced relative to each other and that said connection is releasably lockable.

4. A device as claimed in claim 1 or 3, **characterised in that** the longitudinal carrier (7) is connected with each of the plates P_{A};P_{B} (5;6) by means of a releasably lockable joint (12).

5. A device as claimed in any of the claims 1 to 4, **characterised in that** the longitudinal carrier (7) is itself telescopable, the longitudinal carrier (7) being releasably lockable at any required length.

6. A device as claimed in any of the claims 1 to 5, **characterised in that** the joint (12) is a hinge joint with an axis of rotation extending essentially perpendicularly to the bottom surface (29).

7. A device as claimed in claim 6, **characterised in that** the joint (12) comprises a shaft (57) anchored in the longitudinal carrier (7) which is rotatably mounted in a bore 23 arranged concentrically to the axis of rotation (27) in the plate P_{A} or P_{B} (5;6), respectively.

8. A device as claimed in claim 7, **characterised in that** the shaft (57) is anchored in the longitudinal carrier (7) through non-positive engagement.

9. A device as claimed in claim 7, **characterised in that** the shaft (57) is anchored in the longitudinal carrier (7) through positive engagement.

10. A device as claimed in claim 6, **characterised in that** the joint (12) comprises a shaft (57) anchored concentrically to the axis of rotation (27) in the plate P_{A} or P_{B} (5;6), respectively, which shaft is rotatably mounted in a bore (31) arranged concentrically to the axis of rotation (27) in the longitudinal carrier (7).

11. A device as claimed in claim 10, **characterised in that** the shaft (57) is anchored in the plate P_{A} or P_{B} (5;6), respectively, through non-positive engagement.

12. A device as claimed in claim 12, **characterised in that** the shaft (57) is anchored in the plate P_{A} or P_{B} (5;6), respectively, through positive engagement.

13. A device as claimed in claim 6, **characterised in that** the joint (12) comprises a shaft (57) arranged concentrically to the axis of rotation (27) which is rotatably mounted in the plate P_{A} or P_{B} (5;6), respectively, and in the longitudinal carrier (7).

14. A device as claimed in any of the claims 7 to 14, **characterised in that** the shaft (57) is realised in the form of a sleeve (15), said sleeve (15) being capable of being resiliently spread apart over a certain segment of its length.

15. A device as claimed in claim 14, **characterised in that** a clamping member (13) and a straining means (14) are integrated in the sleeve (15), the clamping member (13) being displaceable relative to the sleeve (15) in a direction parallel to the axis of rotation (27) by means of the straining means (14), so that as a result of the relative displacement of the clamping member (13) the sleeve (15) is spread apart.

16. A device as claimed in claim 15, **characterised in that** the clamping member (13) forms a positive engagement between the longitudinal carrier (7) and the sleeve (15) relative to rotation while being freely movable relative to displacement along the axis of rotation (27).

17. A device as claimed in claim 15, **characterised in that** the clamping member (13) forms a positive engagement between the plate P_{A} or P_{B} (5;6), respectively, and the sleeve (15) relative to rotation while being freely movable relative to displacement along the axis of rotation (27).

18. A device as claimed in claim 14, **characterised in that** two clamping members (13) and a straining means (14) are integrated in the sleeve (15), the clamping members (13) being displaceable relative to each other in a direction parallel to the axis of rotation (27) by the action of the straining means (14), and the sleeve (15) being capable of being spread apart as a result of the displacement of said clamping members (13).

19. A device as claimed in claim 18, **characterised in that** at least one of the clamping members (13) forms a positive engagement between the longitudinal carrier (7) and the sleeve (15) relative to rotation while being freely movable relative to displacement along the axis of rotation (27).

20. A device as claimed in claim 18, **characterised in that** at least one of the clamping members (13) forms a positive engagement between the plate P_{A} or P_{B} (5;6), respectively, and the sleeve (15) relative to rotation while being freely movable relative to displacement along the axis of rotation (27).

21. A device as claimed in any of the claims 15 to 20, **characterised in that** in its spreadable segment the sleeve (15) is slotted along the axis of rotation (27).

22. A device as claimed in any of the claims 15 to 21, **characterised in that** the clamping member(s) (13) is (are) at least partially wedge-shaped.

23. A device as claimed in any of the claims 15 to 22, **characterised in that** the sleeve (15) is at least in part provided with a wedge-shaped slot (32).

24. A device as claimed in claims 22 and 23, **characterised in that** the wedge-like forms of the slot (32) and of the clamping member (13) are complementary.

25. A device as claimed in any of the claims 22 to 24, **characterised in that** the half wedge angle α is between 3 and 20 degrees.

26. A device as claimed in claim 25, **characterised in that** the half wedge angle α is between 5 and 10 degrees.

27. A device as claimed in any of the claims 15 to 26, **characterised in that** the straining means (14) is a threaded bolt.

28. A device as claimed in claim 27, **characterised in that** the threaded bolt is provided with a screw thread (33).

29. A device as claimed in any of the claims 15 to 28, **characterised in that** the clamping member (13) has an internal screw thread (34) complementary to the screw thread (33) of the threaded bolt and that the threaded bolt is mounted within the sleeve (15) in such a way that it is rotatable but axially fixed.

30. A device as claimed in claims 15 and 28, **characterised in that** the sleeve (15) has an internal screw thread complementary to the screw thread (33) and that the threaded bolt is mounted in such a way in the clamping member (13) that it is rotatable but not axially displaceable relative to the clamping member (13).

31. A device as claimed in claims 18 and 28, **characterised in that** one of the clamping members (13) has an internal screw thread complementary to the screw thread (33) of the threaded bolt and that the threaded bolt is mounted in the second clamping member (13) in such a way that the threaded bolt is rotatable but not axially displaceable relative to said clamping member (13) and that the two clamping members (13) are displaced relative to each other along the axis of rotation (27) as the threaded bolt is rotated.

32. A device as claimed in any of the claims 1 to 31, **characterised in that** a displacement of the clamping member (13) causes the sleeve (15) to be expanded in its spreadable segment to such an extent that a non-positive engagement is formed between the sleeve (15) and the surrounding wall of the bore.

33. A device as claimed in any of the claims 1 to 31, **characterised in that** a displacement of the clamping member (13) causes the sleeve (15) to be expanded in its spreadable segment to such an extent that a positive engagement is formed between the sleeve (15) and the surrounding wall of the bore.

34. A device as claimed in claim 33, **characterised in that** said positive engagement consists of complementary toothings (28;51) formed in the resiliently spreadable segment of the sleeve (15) and, correspondingly, on the wall of the bore surrounding the sleeve (15).

35. A device as claimed in claim 34, **characterized in that** the toothings (28;51) have a pitch angle of between 2 and 6 degrees.

36. A device as claimed in claim 35, **characterized in that** the toothings (28;51) have a pitch angle of between 2.5 and 3.5 degrees.

37. A device as claimed in claim 35 or 36, **characterized in that** the tips of the teeth of the toothings (28;51) have an angle of between 60 and 140 degrees.

38. A device as claimed in claim 37, **characterized in that** the tips of the teeth of the toothings (28;51) have an angle of between 90 and 120 degrees.

39. A device as claimed in any of the claims 1 to 38, **characterised in that** the adjustment of the distance between the plates P_{A} (5) and P_{B} (6) is realised by means of a telescopic appliance.

40. A device as claimed in any of the claims 1 to 39, **characterised in that** the plate P_{A} or P_{B} (5;6), respectively, which is connected to the longitudinal carrier (7) by means of the rotation joint (12) is provided with a recess (22) which makes it possible, using a tool, to exert a torsional moment on the device about an axis extending essentially vertically to the bottom surface (29).

41. A device as claimed in any of the claims 1 to 40, **characterised in that** the longitudinal carrier (7) has a recess by means of which, using a tool, a torsional moment about an axis extending essentially vertically to the surface (71) formed by the bottom surfaces (29) of the plates P_{A};P_{B} (5;6) may be exerted on the device.

42. A device as claimed in any of the claims 1 to 39, **characterised in that** the plate P_{A} (5) and the plate P_{B} (6) have each a recess by means of which, using a tool, a torsional moment about an axis extending essentially vertically to the surface (71) formed by the bottom surfaces (29) of the plates P_{A};P_{B} (5;6) may be exerted on the device.

43. A device as claimed in any of the claims 1 to 42, **characterised in that** it further comprises at least two bone screws (8;9;10;11;41;42;73) and that each of the plates P_{A};P_{B} (5;6) is connected to a body K_{A};K_{B} by means of at least one bone screw (8;9;10;11;41;42;73).

44. A device as claimed in any of the claims 1 to 43, **characterised in that** it further comprises at least two bone screws (8;9;10;11;41;42;73) and that for each of the plates P_{A};P_{B} (5;6) at least one bone screw (42;73) is releasably lockable at any given angle of up to ± 20 degrees relative to a perpendicular to the bottom surface (29).

45. A device as claimed in claim 44, **characterised in that** for each of the plates P_{A};P_{B} (5;6) at least one bone screw (42;73) is releasably lockable at any given angle ranging between ± 15 and ± 18 degrees relative to a perpendicular to the bottom surface (29).

46. A device as claimed in claim 45, **characterised in that** the through hole (38;74) for the angularly adjustable bone screw (42) formed in the plates P_{A};P_{B} (5;6) includes a calotte-shaped segment (46).

47. A device as claimed in claim 46, **characterised in that** the calotte-shaped segment (46) of the through hole (38) has a roughened surface.

48. A device as claimed in claim 46, **characterised in that** the calotte-shaped segment (46) of the through hole (38) has a structured surface.

49. A device as claimed in claim 46, **characterised in that** the calotte-shaped segment (46) of the through hole (38) has a toothed surface.

50. A device as claimed in any of the claims 46 to 49, **characterised in that** the bone screw (42) has a slotted, radially resiliently deformable screw head (62) shaped in the form of a spherical segment.

51. A device as claimed in claim 50, **characterised in that** the spherical segment-like screw head (62) of the bone screw (42) has an accommodation for receiving a straining member (66) for spreading apart the spherical segment-like, slotted screw head (62).

52. A device as claimed in claim 51, **characterised in that** the receiving accommodation consists of an inner cone divergent towards the opening of the bore and, further down and adjacent thereto, of an internal screw thread, and that the straining member is a screw having an external thread complementary to said internal screw thread and a male taper complementary to said inner cone.

53. A device as claimed in claim 51, **characterised in that** the receiving accommodation consists of a conical, internal screw thread (64) divergent towards the opening of the bore and that the straining member (66) is a screw having an external screw thread complementary to said internal screw thread (64).

54. A device as claimed in any of the claims 46 to 49, **characterised in that** a slotted collet chuck (45) shaped in the form of a spherical segment is integrated in the calotte-shaped segment (46).

55. A device as claimed in claim 54, **characterised in that** the spherical segment-like collet chuck (45) consists of a softer material than that of the plates P_{A};P_{B} (5;6).

56. A device as claimed in claim 54, **characterised in that** the collet chuck (45) has a structured surface.

57. A device as claimed in claim 56, **characterised in that** the collet chuck (45) has a toothed surface.

58. A device as claimed in claim 54, **characterised in that** the collet chuck (45) has a roughened surface.

59. A device as claimed in any of the claims 54 to 58, **characterised in that** the collet chuck (45) has a through hole (29) extending in the direction of the longitudinal axis (75) of the bone screw (73) and having an inner cone divergent towards the bottom surface (29), the half cone angle being between 3 and 10 degrees.

60. A device as claimed in claim 59, **characterised in that** the half cone angle is between 3 and 5 degrees.

61. A device as claimed in claim 59 or 60, **characterised in that** in the area of the collet chuck (45) the bone screw (73) has an essentially complementary male taper.

62. A device as claimed in claim 61, **characterised in that** in the end portion of said male taper, the bone screw (73) has an accommodation for receiving a straining means for chucking the male taper in the inner cone of the collet chuck (45).

63. A device as claimed in claim 62, **characterised in that** the receiving accommodation consists essentially of an internal screw thread (47) while the straining means consists of a screw (48) having an external screw thread complementary to said internal screw thread (47) and a head diameter greater than the smallest diameter of the inner cone of the collet chuck (45).

64. A device as claimed in claim 62, **characterised in that** the receiving accommodation consists essentially of an external screw thread while the straining means consists of a nut having an internal screw thread complementary to said external screw thread and an outside diameter greater than the smallest diameter of the inner cone of the collet chuck (45).

65. A device as claimed in claim 61, **characterised in that** the collet chuck (45) has an internal screw thread and the bone screw (73) a complementary external screw thread.

66. A device as claimed in any of the claims 43 to 65, **characterised in that** for each of the plates P_{A};P_{B} (5;6) at least one bone screw (41) is releasably lockable at a predefined angle of between 70 and 90 degrees relative to the bottom surface (29) of the plates P_{A};P_{B} (5;6).

67. A device as claimed in claim 66, **characterised in that** for each of the plates P_{A};P_{B} (5;6) at least one bone screw (41) is releasably lockable at a predefined angle of between 80 and 90 degrees relative to the bottom surface (29) of the plates P_{A};P_{B} (5;6).

68. A device as claimed in claim 66 or 67, **characterised in that** the bone screw (41) insertable at a predefined angle relative to the plate P_{A};P_{B} (5;6) has an external screw thread formed in its area of contact with the plate P_{A};P_{B} (5;6) which is tightened against the complementary, internal thread (43) formed in the bore (37) of the plate P_{A};P_{B} (5;6).

69. A device as claimed in any of the claims 43 to 68, **characterised in that** for each of the plates P_{A};P_{B} (5;6) at least one bone screw (42) is insertable at any required angle of between 60 and 90 degrees relative to the bottom surface (29).

70. A device as claimed in claim 69, **characterised in that** for each of the plates P_{A};P_{B} (5;6) at least one bone screw (42) is insertable at any required angle of between 70 and 90 degrees relative to the bottom surface (29).

71. A device as claimed in any of the claims 15 to 21 and 27 to 70, **characterised in that** the clamping member(s) (13) is (are) at least partially conical.

72. A device as claimed in any of the claims 15 to 21 and 27 to 71, **characterised in that** the sleeve (15) is at least in part provided with an inner cone.

73. A device as claimed in claims 71 and 72, **characterised in that** the conical forms present in the sleeve (15) and on the clamping member (13) are complementary.

74. A device as claimed in any of the claims 71 to 73, **characterised in that** the half cone angle α is between 3 and 20 degrees.

75. A device as claimed in claim 74, **characterised in that** the half cone angle α is between 5 and 10 degrees.

76. A device as claimed in claim 1, **characterised in that** the sleeve (15) is anchored in the longitudinal carrier (7).

77. A device as claimed in claim 1, **characterised in that** the longitudinal carrier (7) is connected telescopably displaceable and angularly stable with the other plate P_{A};P_{B} (5;6).

## Revendications

1. Dispositif de liaison d'un corps K_{A} à un corps K_{B}, en particulier de deux os ou fragments d'os, comprenant
A) deux plaques P_{A}; P_{B} (5 ; 6), qui présentent chacune une face inférieure (29) tournée vers le corps K_{A} ; K_{B} à relier, une face supérieure (52) et au moins un trou (37 ; 38) traversant la plaque P_{A} ; P_{B} (5 ; 6) à un angle compris entre 60° et 90° par rapport à la face inférieure (29), pour la réception d'un élément d'ancrage (80) au moyen duquel chacune des plaques P_{A} ; P_{B} (5;6) peut être fixée sur un corps K_{A} ; K_{B} correspondant ;
B) un longeron (7), au moyen duquel les deux plaques P_{A} ; P_{B} (5 ; 6) peuvent être reliées de manière à ce que les faces inférieures (29) des plaques P_{A} ; P_{B} (5 ; 6) se trouvent dans un plan commun (71) et que la distance entre les deux plaques P_{A} ; P_{B} (5 ; 6) puisse être ajustée parallèlement au plan commun (71), dans lequel
C) le longeron (7) est relié à l'une des plaques P_{A} ; P_{B} (5 ; 6) par l'intermédiaire d'une articulation (12) pouvant être bloquée de manière amovible,
**caractérisé en ce que**
D) l'articulation (12) est réalisée sous forme d'articulation à charnière et comprend un fourreau (15) pouvant être expansé de manière élastique sur au moins une partie de sa longueur, au moins un élément de serrage (13) et un moyen de blocage (14), dans laquelle le au moins un élément de serrage (13) est mobile par rapport au fourreau (15) à l'aide du moyen de blocage (14).

2. Dispositif selon la revendication 1, **caractérisé en ce que** le longeron (7) est relié d'un côté à l'une des plaques P_{A}; P_{B} (5 ; 6) par l'intermédiaire d'une articulation (12) pouvant être bloquée de manière amovible.

3. Dispositif selon la revendication 2, **caractérisé en ce que** la liaison entre le longeron (7) et l'une des plaques P_{A} ; P_{B} (5 ; 6) permet un déplacement entre le longeron (7) et la plaque P_{A} ; P_{B} (5 ; 6) et la liaison peut être bloquée de manière amovible.

4. Dispositif selon la revendication 1 ou 3, **caractérisé en ce que** le longeron (7) est relié à chacune des plaques P_{A} ; P_{B} (5 ; 6) par l'intermédiaire d'une articulation (12) pouvant être bloquée de manière amovible.

5. Dispositif selon l'une quelconque des revendications 1 à 4, **caractérisé en ce que** le longeron (7) est lui-même télescopable, dans lequel la longueur du longeron (7) peut être fixée de manière amovible.

6. Dispositif selon l'une quelconque des revendications 1 à 5, **caractérisé en ce que** l'articulation (12) est une articulation à charnière ayant un axe de rotation (27) essentiellement perpendiculaire à la face inférieure (29).

7. Dispositif selon la revendication 6, **caractérisé en ce que** l'articulation (12) comprend une broche (57), ancrée dans le longeron (7), qui est disposée de manière à pouvoir tourner dans un alésage (23) réalisé dans la plaque P_{A} ou P_{B} (5 ; 6) concentriquement à l'axe de rotation (27).

8. Dispositif selon la revendication 7, **caractérisé en ce que** la broche (57) est ancrée par enfoncement forcé dans le longeron (7).

9. Dispositif selon la revendication 7, **caractérisé en ce que** la broche (57) est ancrée par emboîtement dans le longeron (7).

10. Dispositif selon la revendication 6, **caractérisé en ce que** l'articulation (12) comprend une broche (57), ancrée dans la plaque P_{A} ou P_{B} (5 ; 6) concentriquement à l'axe de rotation (27), laquelle est disposée de manière à pouvoir tourner dans un alésage (31) réalisé dans le longeron (7) concentriquement à l'axe de rotation (27).

11. Dispositif selon la revendication 10, **caractérisé en ce que** la broche (57) est ancrée par enfoncement forcé dans la plaque P_{A} ou P_{B} (5 ; 6).

12. Dispositif selon la revendication 10, **caractérisé en ce que** la broche (57) est ancrée par emboîtement dans la plaque P_{A} ou P_{B} (5 ; 6).

13. Dispositif selon la revendication 6, **caractérisé en ce que** l'articulation (12) comprend une broche (57), disposée concentriquement à l'axe de rotation (27), laquelle est disposée de manière à pouvoir tourner dans la plaque P_{A} ou P_{B} (5 ; 6) et dans le longeron (7).

14. Dispositif selon l'une quelconque des revendications 7 à 13, **caractérisé en ce que** la broche (57) est réalisée sous forme de fourreau (15), dans lequel le fourreau (15) peut être expansé de manière élastique sur une certaine zone de sa longueur.

15. Dispositif selon la revendication 14, **caractérisé en ce qu'**un élément de serrage (13) et un moyen de blocage (14) sont intégrés dans le fourreau (15), dans lequel l'élément de serrage (13) est mobile à l'aide du moyen de blocage (14) par rapport au fourreau (15) parallèlement à l'axe de rotation (27), de sorte que le fourreau (15) est expansé du fait du mouvement relatif de l'élément de serrage (13).

16. Dispositif selon la revendication 15, **caractérisé en ce que** l'élément de serrage (13), pour ce qui est de la rotation, forme une liaison par emboîtement entre le longeron (7) et le fourreau (15) et, pour ce qui est du déplacement axial, est librement mobile le long de l'axe de rotation (27).

17. Dispositif selon la revendication 15, **caractérisé en ce que** l'élément de serrage (13), pour ce qui est de la rotation, forme une liaison par emboîtement entre la plaque P_{A} ou P_{B} (5 ; 6) et le fourreau (15) et, pour ce qui est du déplacement axial, est librement mobile le long de l'axe de rotation (27).

18. Dispositif selon la revendication 14, **caractérisé en ce que** deux éléments de serrage (13) et un moyen de blocage (14) sont intégrés dans le fourreau (15), dans lequel les éléments de serrage (13) sont mobiles l'un par rapport à l'autre à l'aide du moyen de blocage (14) le long de l'axe de rotation (27), et le fourreau (15) peut être expansé du fait de ce mouvement des éléments de serrage (13).

19. Dispositif selon la revendication 18, **caractérisé en ce qu'**au moins un des éléments de serrage (13), pour ce qui est de la rotation, forme une liaison par emboîtement entre le longeron (7) et le fourreau (15) et, pour ce qui est du déplacement axial, est librement mobile le long de l'axe de rotation (27).

20. Dispositif selon la revendication 18, **caractérisé en ce qu'**au moins un des éléments de serrage (13), pour ce qui est de la rotation, forme une liaison par emboîtement entre la plaque P_{A} ou P_{B} (5 ; 6) et le fourreau (15) et, pour ce qui est du déplacement axial, est librement mobile le long de l'axe de rotation (27).

21. Dispositif selon l'une quelconque des revendications 15 à 20, **caractérisé en ce que** le fourreau (15), dans la zone pouvant être expansée, est fendu le long de l'axe de rotation (27).

22. Dispositif selon l'une quelconque des revendications 15 à 21, **caractérisé en ce que** le ou les éléments de serrage (13) sont réalisés au moins en partie en forme de coin.

23. Dispositif selon l'une quelconque des revendications 15 à 22, **caractérisé en ce que** le fourreau (15) est pourvu au moins en partie d'une fente cunéiforme (32).

24. Dispositif selon les revendications 22 et 23, **caractérisé en ce que** les formes de coin de la fente (32) et de l'élément de serrage (13) sont réalisées de manière complémentaire.

25. Dispositif selon l'une quelconque des revendications 22 à 24, **caractérisé en ce que** les formes de coin de la fente (32) et de l'élément de serrage (13) présentent un demi angle de conicité α compris entre 3° et 20°.

26. Dispositif selon la revendication 25, **caractérisé en ce que** le demi angle de conicité α est compris entre 5° et 10°.

27. Dispositif selon l'une quelconque des revendications 15 à 26, **caractérisé en ce que** le moyen de blocage (14) est un boulon fileté.

28. Dispositif selon la revendication 27, **caractérisé en ce que** le boulon fileté présente un filetage (33).

29. Dispositif selon les revendications 15 et 28, **caractérisé en ce que** l'élément de serrage (13) présente un taraudage (34) complémentaire au filetage (33) du boulon fileté et le boulon fileté est disposé de manière à pouvoir tourner mais de manière fixe sur l'axe dans le fourreau (15).

30. Dispositif selon les revendications 15 et 28, **caractérisé en ce que** le fourreau (15) présente un taraudage complémentaire au filetage (33) et le boulon fileté est disposé dans l'élément de serrage (13) de telle manière qu'il peut tourner mais ne peut pas être déplacé axialement par rapport à l'élément de serrage (13).

31. Dispositif selon les revendications 18 et 28, **caractérisé en ce que** l'un des éléments de serrage (13) présente un taraudage complémentaire au filetage (33) du boulon fileté et le boulon fileté est disposé dans le deuxième élément de serrage (13) de telle manière que le boulon fileté peut tourner mais ne peut pas être déplacé axialement par rapport à l'élément de serrage (13) en question et que lors d'une rotation du boulon fileté, les deux éléments de serrage (13) se déplacent l'un par rapport à l'autre le long de l'axe de rotation (27).

32. Dispositif selon l'une quelconque des revendications 1 à 31, **caractérisé en ce que** lors d'un déplacement de l'élément de serrage (13), le fourreau (15), dans la zone pouvant être expansée, est tellement expansé que cela provoque une liaison par enfoncement forcé entre le fourreau (15) et la paroi de l'alésage qui l'entoure.

33. Dispositif selon l'une quelconque des revendications 1 à 31, **caractérisé en ce que** lors d'un déplacement de l'élément de serrage (13), le fourreau (15), dans la zone pouvant être expansée, est tellement expansé que cela provoque une liaison par emboîtement entre le fourreau (15) et la paroi environnante de l'alésage.

34. Dispositif selon la revendication 33, **caractérisé en ce que** la liaison par emboîtement se compose de dentures complémentaires (28 ; 51), lesquelles sont disposées au niveau de la zone du fourreau (15) pouvant être expansée de manière élastique et de manière correspondante au niveau de la paroi de l'alésage entourant le fourreau (15).

35. Dispositif selon la revendication 34, **caractérisé en ce que** les dentures (28 ; 51) présentent un pas angulaire compris entre 2° et 6°.

36. Dispositif selon la revendication 35, **caractérisé en ce que** les dentures (28 ; 51) présentent un pas angulaire compris entre 2,5° et 3,5°.

37. Dispositif selon la revendication 35 ou 36, **caractérisé en ce que** les pointes de dent des dentures (28 ; 51) présentent un angle compris entre 60° et 140°.

38. Dispositif selon la revendication 37, **caractérisé en ce que** les pointes de dent des dentures (28 ; 51) présentent un angle compris entre 90° et 120°.

39. Dispositif selon l'une quelconque des revendications 1 à 38, **caractérisé en ce que** le réglage de la distance entre les plaques P_{A} (5) et P_{B} (6) est de type télescopique.

40. Dispositif selon l'une quelconque des revendications 1 à 39, **caractérisé en ce que** la plaque P_{A} ou P_{B} (5 ; 6) reliée au longeron (7) par l'intermédiaire de l'articulation pivotante (12) présente un évidement (22), par l'intermédiaire duquel un couple de torsion autour d'un axe essentiellement perpendiculaire à la face inférieure (29) peut être exercé sur le dispositif à l'aide d'un outil.

41. Dispositif selon l'une quelconque des revendications 1 à 40, **caractérisé en ce que** le longeron (7) présente un évidement, par l'intermédiaire duquel un couple de torsion autour d'un axe essentiellement perpendiculaire à la surface (71) définie par les faces inférieures (29) des plaques P_{A}; P_{B} (5 ; 6) peut être exercé sur le dispositif à l'aide d'un outil.

42. Dispositif selon l'une quelconque des revendications 1 à 39, **caractérisé en ce que** la plaque P_{A} (5) et la plaque P_{B} (6) présentent chacune un évidement, par l'intermédiaire duquel un couple de torsion autour d'un axe essentiellement perpendiculaire à la surface (71) définie par les faces inférieures (29) des plaques P_{A}; P_{B} (5 ; 6) peut être exercé sur le dispositif à l'aide d'un outil.

43. Dispositif selon l'une quelconque des revendications 1 à 42, **caractérisé en ce qu'**il comprend en outre au moins deux vis à os (8 ; 9 ; 10 ; 11 ; 41 ; 42 ; 73) et chacune des plaques P_{A}; P_{B} (5 ; 6) peut être reliée à un corps K_{A} ; K_{B} par l'intermédiaire d'au moins une vis à os (8;9; 10; 11 ; 41 ; 42 ; 73).

44. Dispositif selon l'une quelconque des revendications 1 à 43, **caractérisé en ce qu'**il comprend en outre au moins deux vis à os (8 ; 9 ; 10 ; 11 ; 41 ; 42 ; 73) et **en ce que**, par plaque P_{A}; P_{B} (5 ; 6), au moins une vis à os (42 ; 73) peut être bloquée de manière amovible à tout angle souhaité jusqu'à ± 20° par rapport à la perpendiculaire de la face inférieure (29).

45. Dispositif selon la revendication 44, **caractérisé en ce que**, par plaque P_{A}; P_{B} (5 ; 6), au moins une vis à os (42; 73) peut être bloquée de manière amovible à tout angle compris entre ± 15° et ± 18° par rapport à la perpendiculaire de la face inférieure (29).

46. Dispositif selon la revendication 45, **caractérisé en ce que** le trou traversant (38 ; 74) pour la vis à os (42) à angle réglable dans les plaques P_{A} ; P_{B} (5 ; 6) présente un segment (46) réalisé en forme de calotte.

47. Dispositif selon la revendication 46, **caractérisé en ce que** le segment en forme de calotte (46) du trou traversant (38) présente une surface rugueuse.

48. Dispositif selon la revendication 46, **caractérisé en ce que** le segment en forme de calotte (46) du trou traversant (38) présente une surface texturée.

49. Dispositif selon la revendication 46, **caractérisé en ce que** le segment en forme de calotte (46) du trou traversant (38) présente une surface dentée.

50. Dispositif selon l'une quelconque des revendications 46 à 49, **caractérisé en ce que** la vis à os (42) présente une tête de vis (62) en forme de segment sphérique, fendue et à déformation radiale élastique.

51. Dispositif selon la revendication 50, **caractérisé en ce que** la tête de vis en forme de segment sphérique (62) de la vis à os (42) présente un logement pour un élément de blocage (66) pour expanser la tête de vis fendue en forme de segment sphérique (62).

52. Dispositif selon la revendication 51, **caractérisé en ce que** le logement se compose d'un cône interne divergeant vers l'ouverture et d'un taraudage adjacent dans la profondeur, et l'élément de blocage est une vis avec un filetage complémentaire au taraudage et un cône externe complémentaire au cône interne.

53. Dispositif selon la revendication 51, **caractérisé en ce que** le logement se compose d'un taraudage conique (64) divergeant vers l'ouverture et l'élément de blocage (66) est une vis avec un filetage complémentaire au taraudage (64).

54. Dispositif selon l'une quelconque des revendications 46 à 49, **caractérisé en ce qu'**une pince de serrage (45) fendue en forme de segment sphérique est intégrée dans le segment en forme de calotte (46).

55. Dispositif selon la revendication 54, **caractérisé en ce que** la pince de serrage (45) en forme de segment sphérique se compose d'un matériau plus mou que la plaque P_{A}; P_{B} (5 ; 6).

56. Dispositif selon la revendication 54, **caractérisé en ce que** la pince de serrage (45) présente une surface texturée.

57. Dispositif selon la revendication 56, **caractérisé en ce que** la pince de serrage (45) présente une surface dentée.

58. Dispositif selon la revendication 54, **caractérisé en ce que** la pince de serrage (45) présente une surface rugueuse.

59. Dispositif selon l'une quelconque des revendications 54 à 58, **caractérisé en ce que** la pince de serrage (45) présente un trou traversant (76) s'étendant dans le sens de l'axe longitudinal (75) de la vis à os (73), divergeant de manière conique vers la face inférieure (29), dans lequel le demi angle de cône est compris entre 3° et 10°.

60. Dispositif selon la revendication 59, **caractérisé en ce que** le demi angle de cône est compris entre 3° et 5°.

61. Dispositif selon la revendication 59 ou 60, **caractérisé en ce que** la vis à os (73) présente, dans la zone de la pince de serrage (45), un cône externe essentiellement complémentaire.

62. Dispositif selon la revendication 61, **caractérisé en ce que** la vis à os (73) présente, à l'extrémité du cône externe, un logement destiné à un moyen de blocage pour bloquer le cône externe dans le cône interne de la pince de serrage (45).

63. Dispositif selon la revendication 62, **caractérisé en ce que** le logement se compose essentiellement d'un taraudage (47) et le moyen de blocage d'une vis (48) ayant un filetage complémentaire au taraudage (47) et un diamètre de tête plus grand que le plus petit diamètre du cône interne de la pince de serrage (45).

64. Dispositif selon la revendication 62, **caractérisé en ce que** le logement se compose essentiellement d'un filetage et le moyen de blocage d'un écrou ayant un taraudage complémentaire au filetage et un diamètre extérieur plus grand que le plus petit diamètre du cône interne de la pince de serrage (45).

65. Dispositif selon la revendication 61, **caractérisé en ce que** la pince de serrage (45) présente un taraudage et la vis à os (73) un filetage complémentaire.

66. Dispositif selon l'une quelconque des revendications 43 à 65, **caractérisé en ce que**, par plaque P_{A}; P_{B} (5 ; 6), au moins une vis à os (41) peut être bloquée de manière amovible à un angle prédéfini compris entre 70° et 90° par rapport à la face inférieure (29) des plaques P_{A} ; P_{B} (5 ; 6).

67. Dispositif selon la revendication 66, **caractérisé en ce que**, par plaque P_{A};P_{B} (5 ; 6), au moins une vis à os (41) peut être bloquée de manière amovible à un angle prédéfini compris entre 80° et 90° par rapport à la face inférieure (29) des plaques P_{A}; P_{B} (5 ; 6).

68. Dispositif selon la revendication 66 ou 67, **caractérisé en ce que** la vis à os (41) pouvant être insérée à un angle prédéfini par rapport à la plaque P_{A}; P_{B} (5 ; 6) présente, dans la zone de contact avec la plaque P_{A}; P_{B} (5 ; 6), un filetage qui est bloqué contre le taraudage (43) complémentaire se trouvant dans l'alésage (37) de la plaque P_{A}; P_{B} (5 ; 6).

69. Dispositif selon l'une quelconque des revendications 43 à 68, **caractérisé en ce que**, par plaque P_{A}; P_{B} (5 ; 6), au moins une vis à os (42) peut être insérée à tout angle compris entre 60° et 90° par rapport à la face inférieure (29).

70. Dispositif selon la revendication 69, **caractérisé en ce que**, par plaque P_{A}; P_{B} (5 ; 6), au moins une vis à os (42) peut être insérée à tout angle compris entre 70° et 90° par rapport à la face inférieure (29).

71. Dispositif selon l'une quelconque des revendications 15 à 21 et 27 à 70, **caractérisé en ce que** le ou les éléments de serrage (13) sont réalisés au moins en partie en forme de cône.

72. Dispositif selon l'une quelconque des revendications 15 à 21 et 27 à 71, **caractérisé en ce que** le fourreau (15) est pourvu au moins en partie d'un cône interne.

73. Dispositif selon les revendications 71 et 72, **caractérisé en ce que** les formes de cône dans le fourreau (15) et de l'élément de serrage (13) sont réalisées de manière complémentaire.

74. Dispositif selon l'une quelconque des revendications 71 à 73, **caractérisé en ce que** les formes de cône dans le fourreau (15) et de l'élément de serrage (13) présentent un demi angle de cône β compris entre 3° et 20°.

75. Dispositif selon la revendication 74, **caractérisé en ce que** le demi angle de cône β est compris entre 5° et 10°.

76. Dispositif selon la revendication 1, **caractérisé en ce que** le fourreau (15) est ancré dans le longeron (7).

77. Dispositif selon la revendication 1, **caractérisé en ce que** le longeron (7) est relié à l'autre plaque P_{A} ; P_{B} (5 ; 6) de manière à pouvoir être déplacé de manière télescopique et à angle fixe.
